# EUROPEAN PATENT SPECIFICATION

(11) **EP 2 468 204 B1**
(45) Date of publication and mention of the grant of the patent: **27.07.2016**
(21) Application number: 11195045.7
(22) Date of filing: 21.12.2011
(51) Int. Cl.: A61B 18/02

(54) **Cryogenic fluid dispensing device**
Vorrichtung zur Abgabe von kryogenischer Flüssigkeit
Appareil de distribution de liquide cryogénique

(30) Priority: 21.12.2010 US 974430
(43) Date of publication of application: 27.06.2012
(73) Proprietor: Cryoconcepts L.P., Bethlehem, Pennsylvania 18015 (US)
(72) Inventor: Niedbala, Sam R., Allentown, Pennsylvania 18103 (US); Young, Lincoln C., Ithaca, New York 14850 (US); Zhou, Peng, Newtown, Pennsylvania 18940 (US)
(74) Representative: Molnia, David

(56) References cited:
- EP-A2- 2 292 170
- WO-A2-2011/005495
- GB-A- 1 402 632
- US-A- 3 439 680
- US-A- 3 536 075
- US-A- 3 993 075
- US-A- 4 201 319
- US-A1- 2004 102 768
- US-A1- 2009 270 851

## Description

### TECHNICAL FIELD

The present teachings relate to devices and methods for dispensing a cryogenic fluid. In particular, the present teachings relate to cryosurgical devices and methods for cooling surfaces by either directly dispensing a cryogenic fluid to a surface to be treated or by dispensing a cryogenic fluid onto an applicator to be placed in contact with a surface to be treated.

### BACKGROUND

A number of procedures have been developed for treating superficial lesions, such as, for example, warts, on human and animal skin. Lesions can be removed, for example, through the localized freezing of the skin lesion tissue by a cooling fluid, such as a liquid refrigerant. Physicians have used liquid nitrogen applications, for example, to freeze and remove lesions from a patient's skin. Conventional methods of treatment, however, may have the disadvantages of requiring specialized equipment to condense the nitrogen gas, the need for specialized storage devices, and the inherent hazards of handling and dispensing materials having very low boiling points, for example, as low as approximately -196° C in the case of liquid nitrogen.

Cryosurgery is used by medical professionals to treat a variety of lesions. Extreme cold works to destroy tissue through lysis of cells. This may occur through the formation of ice or rapid changes in osmotic pressure. Both can work to increase the overall effectiveness of cryosurgical treatments.

More recently, various methods have been developed to treat skin lesions cryosurgically by employing a cooling fluid (e.g., a cryogenic fluid) contained, for example, in a handheld pressurized container. Such cryosurgical devices generally rely upon a liquefied (compressed) gas, such as, for example, butane, propane, or dimethyl ether (DME), to rapidly cool an applicator tip or "bud" based on the principles of "heat of vaporization." In other words, as the compressed gas flows to and contacts a surface of an applicator, such as, for example, a porous applicator bud, rapid evaporation of the gas causes the applicator surface to cool to temperatures which are lower than the temperature of the liquefied gas alone. In several such methods, an effective amount of the cryogenic fluid from the pressurized container can be dispensed, for example, into a hollow supply tube having a cotton, fiber and/or plastic foam bud located at the distal end of the supply tube. The cryogenic fluid accumulates in the applicator and upon evaporation, cools the applicator to temperatures well below freezing. The applicator can be placed in contact with the skin surface of the lesion for a period of time sufficient to reduce the temperature of the skin lesion tissue to temperatures that freeze the skin, such that permanent, irreversible rupture of the cellular membranes of the tissue occurs.

Cryosurgical devices currently utilizing the heat of vaporization principal in combination with compressed gases, such as DME for example, can pose various issues. For example, the devices can depend significantly upon the particular gas used and rates of evaportion from the applicator may be relatively long (e.g., on the order of 15-30 seconds). Moreover, the effective temperature of the applicator (i.e., the temperature of the applicator that is sufficient to cause freezing of the skin lesion) may be reached for only a short period of time, particularly once placed in contact with the warmer surface of the skin lesion, thereby limiting effective freezing of the target tissue.

Various additional cryosurgical devices can utilize liquid nitrogen, or other liquefied gases such as, for example, chlorofluoro carbons or nitrous oxide, which have significantly lower boiling points and thus can be dispensed at colder temperatures than some conventional "heat of vaporization" gases such as DME, thereby achieving more aggressive freezing effects. Such cryosurgical devices, however, are generally still relatively complex in their structure, using complicated volving mechanisms and dispensers to deliver the liquefied gas. Accordingly, problems can arise with such devices due to the high pressures exhibited by the gases, the complicated manner in which the cryogenic fluid is moved from the container to the dispensing tip, the ease of use, and/or the cost associated with manufacture and/or assembly of the devices.

Accordingly, it may be desirable to provide a cryosurgical device that is both simple in terms of structure and use, and capable of delivering a variety of cryogenic fluids, including more aggressive cooling agents, such as, for example, nitrous oxide and liquid nitrogen, in an amount sufficient to achieve effective cryosurgical treatment. It may be further desirable to provide a disposable cryosurgical device that can be discarded once spent. It may, therefore, be desirable to provide an economical device with simpler structural components and flow regulation mechanisms, which can also reduce waste of the cryogenic fluid as it is moved from a container, for example to an applicator, for dispensing at a desired location.

US 3,993,075 A describes a disposable, defrostable cryosurgical probe. US 4,201,319 A describes a dispensing system employing liquid cryogen, GB 1 402 632 A describes a hand-held cryogenic probe. WO 20111005495 A2, which is prior art according to Article 54(3) EPC, describes devices and methods for dispensing a cryogenic fluid.

US 3,993,075 A pertains to a disposable, defrostable cryosurgical probe.

US 4,201,319 A pertains to a dispensing system employing liquid cryogen.

GB 1 402 632 A pertains to improvements in and relating to medical equipment.

US 3,536,075 A pertains to a cryosurgical instrument.

US 3,439,680 A pertains to a surgical instrument for cataract removal.

### SUMMARY

The present teachings may solve one or more of the above-mentioned problems and/or may demonstrate one or more of the above-mentioned desirable features. Other features and/or advantages may become apparent from the description that follows.

The invention is defined in claim 1. Preferable embodiments are included in the dependent claims as well the specification.

One example includes a self-contained disposable cryosurgical device that sprays a stream of liquid nitrous oxide. Evaporation of the liquefied nitrous oxide gas draws heat from the surroundings. The self-contained disposable cryosurgical device serves as a reservoir for the cryogen-N20, delivering the liquid gas directly onto a lesion to be treated at -89° C. Following cryo treatment, necrosis of the site can occur. Recovery takes about 10 to 14 days, with new tissue growing inwards from the surrounding epidermis and the more deeply situated adnexa. The lesions that can be treated include genital lesions, molluseum contaglosum, seborrheic keratoses, skin tags, verruca plantaris, verruca vulgaris, verruca plans, actinic keratosis, lentigo, and the like.

In accordance with various exemplary embodiments of the present teachings, a dispensing head for dispensing a cryogenic fluid may comprise a flow passage configured to be placed in flow communication with a reservoir containing a cryogenic fluid, the flow passage defining a flow passage inlet opening configured to receive the cryogenic fluid from the reservoir, and a flow passage outlet opening opposite the flow passage inlet opening. The dispensing head may further comprise a dispensing member configured to dispense the cryogenic fluid, the dispensing member defining a lumen having a lumen inlet opening and a lumen outlet opening: and at least one porous member disposed in the flow passage, the at least one porous member being configured as a primary flow regulation mechanism to limit a flow rate of the cryogenic fluid as it flows from the reservoir to the lumen outlet opening.

In accordance with various additional exemplary embodiments of the present teachings, a dispensing head for dispensing a cryogenic fluid may comprise a flow passage configured to be placed in flow communication with a reservoir containing a cryogenic fluid, the flow passage defining a flow passage inlet opening configured to receive the cryogenic fluid from the reservoir, and a flow passage outlet opening opposite the flow passage inlet opening. The dispensing head may further comprise a dispensing member configured to dispense the cryogenic fluid, the dispensing member defining a lumen having a lumen inlet opening and a lumen outlet opening; and an actuation member configured to move between a first position in which the flow passage outlet opening is not aligned and is blocked from flow communication with the lumen inlet opening, and a second position in which the flow passage outlet opening is aligned with and in flow communication with the lumen inlet opening.

In accordance with various further exemplary embodiments of the present teachings, a method for dispensing a cryogenic fluid may comprise coupling a dispensing head defining a flow passage, a dispensing member, an actuation member, and at least one porous member disposed in the flow passage to a container defining a reservoir. The method may further comprise actuating the dispensing head so as to move an inlet opening of the dispensing member from a first position in which an outlet of the flow passage is not aligned and is blocked from flow communication with the dispensing member inlet opening, to a second position in which the flow passage outlet opening is aligned with and in flow communication with the dispensing member inlet opening. The method may further comprise flowing an amount of cryogenic fluid from the reservoir toward the dispensing member inlet opening through the flow passage and dispensing the cryogenic fluid from a dispensing member outlet opening to a target location, wherein a flow rate of the cryogenic fluid flowing from the reservoir to the dispensing member outlet opening is primarily regulated by passing the cryogenic fluid through at least one porous member.

In accordance with various additional exemplary embodiments of the present teachings, a method for dispensing a cryogenic fluid may comprise placing an application member in contact with a skin surface and supplying a cryogenic fluid to the application member while the application member is in contact with the skin surface. The method may further comprise diffusing the cryogenic fluid through the application member to the skin surface at a temperature that is directly effective to freeze the skin surface such that permanent, irreversible rupture of cellular membranes of cells of the skin surface occurs while the cryogenic fluid is being delivered to the skin surface.

One example includes a method for dispensing a cryogenic fluid including opening an activation lever on the cryogen dispensing device to access a cryogenic reservoir compartment, inserting a cryogenic liquid cartridge into the cryogenic reservoir compartment, and returning the activation lever from its extended position to a fully closed position. The method also includes positioning the end of the activation lever into an opening on the device. An audible, tactile, or visual indication can be provided to indicate to a user that the cryogenic liquid cartridge and activation lever are properly seated and positioned on the device, and that the device is ready for use. Similarly, an interlock device can be provided to prevent the activation lever from being moved back from the opening. When fully closed, an eccentric hub on the activation lever displaces the cryogenic liquid cartridge, which is pierced by a pierce pin in the valve assembly to enable the flow of cryogenic material. The cryogen dispensing device can then be used to treat a target lesion. The device is then positioned vertically downwards over the lesion at a distance of approximately 1 cm, and the actuator is activated to initiate the spray of cryogen. In this position, liquid cryogenic material in the cartridge flows through the valve, while gaseous cryogenic material remains in the cartridge. The device can be fixed or moved to cover the lesion area, and the actuator is released to stop the cryogen spray.

In one example of the claimed invention, an activation valve assembly includes a valve body and a valve slide. The valve body includes a flow passage with a flow passage inlet that receives a cryogenic material and a flow passage outlet that delivers the received cryogenic material. The valve slide slidably engages with the valve body. The valve slide includes a lumen with a lumen inlet opening for receiving cryogenic material from the flow passage outlet of the valve body and a lumen outlet opening that delivers the cryogenic material to a target. The target can be a lesion directly, or can be an applicator device, a fluid retaining device, and the like.

The valve slide is engaged with the valve body and is positioned in a dosed position where the lumen inlet opening is offset from the flow passage outlet of the valve body to prevent flow of the cryogenic material. When the cryogenic material is to be delivered to a target, the valve slide is slid along the valve body to engage with the valve body in an open position where the lumen inlet opening of the valve slide is aligned with the flow passage outlet of the valve body to form a flow path to allow the flow of the cryogenic material.

The activation valve assembly can also include a sealing member surrounding the flow passage outlet opening and disposed adjacent the valve slide. For example, the sealing member can be an o-ring or other mechanical gasket. The sealing member can be made using synthetic rubber including Buna rubber, nitrile rubber, styrene-butadiene rubber, nitrile butadiene rubber, or other materials such as silicone, natural rubbers, and the like.

The activation valve assembly can also include a dispensing member disposed in the valve slide. The dispensing member can include the lumen inlet opening and the lumen outlet opening. The dispensing member can be made using polyaryletheretherketone (PEEK), and/or combinations of materials, including, for example, glass, plastic and/or metal and adhesives.

The flow passage inlet opening of the activation valve assembly can include a piercing member. The piercing member can be at least partially disposed in the valve body and configured to puncture a seal on the cryogen reservoir to enable the liquid cryogen to flow to the valve assembly. The piercing member can be a hollow metal member or may be another suitable material configured to puncture the seal of the cryogen reservoir. The piercing member can be configured to puncture the seal of the cryogen reservoir at a right-angle, or can be configured to puncture the seal at an angle, for example using a beveled tip on the piercing member.

The activation valve assembly can also include a filter in the valve body disposed in the flow passage between the flow passage inlet opening and the flow passage outlet opening. The filter can be cylindrical in shape or can be disk-shaped, for example, as well as other shapes suitable to provide a porous member in the flow path of the cryogenic fluid. The filter can provide flow regulation as well as filtration of contaminants or other materials from the cryogenic material,

The filter can be made from a variety of materials depending upon the type, amount, and target of the cryogenic fluid. For example, the filter can include high solid filtration and separation materials, including polyethylene substrates, polyvinylidene difluoride (PVDF) membranes, sintered thermoplastic or metals, and the like.

The activation valve assembly can also include a sealing member surrounding the flow passage inlet opening. The sealing member surrounding the flow passage inlet opening can be an a-ring or other mechanical gasket. The sealing member can be made using synthetic or natural rubbers including Buna rubber, nitrile rubber, styrene-butadiene rubber, nitrile butadiene rubber, or other materials such as silicone, natural rubbers, and the like.

Additional objects and advantages will be set forth in part in the description which follows, and in part will be obvious from the description, or may be learned by practice of the present teachings. At least some of the objects and advantages may be realized and attained by means of the elements and combinations particularly pointed out in the appended claims.

It is to be understood that both the foregoing general description and the following detailed description are exemplary and explanatory only and are not restrictive of the claims; rather, the claims are entitled to their full breadth and scope, including equivalents.

### BRIEF DESCRIPTION OF THE DRAWINGS

The present teachings can be understood from the following detailed description either alone or together with the accompanying drawings. The drawings are included to provide a further understanding, and are incorporated in and constitute a part of this specification. The drawings illustrate one or more exemplary embodiments of the present teachings and together with the description serve to explain various principles and operation.
FIG. 1 is a top perspective view of an exemplary embodiment of a device for dispensing a cryogenic fluid in accordance with the present teachings;
FIG. 2 is a side view of the device of FIG. 1;
FIG. 3 is a top plan view of the device of FIG. 1, showing an actuation member in a first position;
FIG. 4 is a cross-sectional view of the device of FIG. 1, taken along line 4-4 of FIG. 3;
FIG. 5 is an enlarged view of the dispensing head of FIG. 4;
FIG. 6 is a top plan view of the device of FIG. 1, showing the actuation member in a second position;
FIG. 7 is a cross-sectional view of the device of FIG. 1, taken along line 7-7 of FIG. 6;
FIG. 8 is an enlarged view of the dispensing head of FIG. 7;
FIG. 9 is a partial enlarged view of another exemplary embodiment of a dispensing head in accordance with the present teachings;
FIG. 10 is a top perspective view of another exemplary embodiment of a device for dispensing a cryogenic fluid in accordance with the present teachings;
FIG. 11 is a top plan view of the device of FIG. 10;
FIG. 12 is a perspective view of an exemplary embodiment of a dispensing member in accordance with the present teachings;
FIG. 13 is a cross-sectional view of an exemplary embodiment of an applicator in accordance with the present teachings;
FIG. 14 is a cross-sectional view of another exemplary embodiment of an applicator in accordance with the present teachings;
FIG. 15 is a cross-sectional view of another exemplary embodiment of an applicator in accordance with the present teachings;
FIG. 16 is a graph of temperature versus time obtained from experiments using a device in accordance with the present teachings;
FIG. 17 is a graph of temperature versus time obtained from experiments using another device in accordance with the present teachings; and
FIG. 18 is a graph of temperature versus time obtained from experiments using yet another device in accordance with the present teachings.
FIGS. 19A-B are views of a cryogenic dispensing device in accordance with the claimed invention.
FIG. 20 is an exploded view of a cryogenic dispensing device in accordance with the claimed invention.
FIG. 21 is a perspective view of a slide valve assembly used in a cryogenic dispensing device in accordance with the claimed invention.
FIG. 22 is an exploded view of a slide valve assembly used in a cryogenic dispensing device in accordance with the claimed invention.
FIG. 23 is a close-up view of a pierce pin used in a slide valve assembly in accordance with the claimed invention.

### DESCRIPTION OF EXEMPLARY EMBODIMENTS

The present teachings contemplate devices that are both relatively simple in structure and use, and capable of dispensing cryogenic fluid at sufficiently cold temperatures and pressures for a sufficient period of time so as to effect cryosurgical treatment. Devices and methods in accordance with the present teachings may dispense, for example, a sufficient and substantially uniform amount of a cryogenic fluid to an applicator to be placed in contact with a target freeze area, such as, for example, the tissue of a skin lesion. Devices in accordance with the present teachings may effectively treat and/or remove various types of skin lesions, including but not limited to, for example, verruca (warts), keratoses, achrocordon, molluscum contagiosum, age spots, dermatofibroma, keloids, granuloma, annulare, porokeratosis plantaris, angioman, lentigo discreta, chondrodermatitis, epithelial nevus, leokoplakia, granuloma pyogenicuni, and/or kaposi's sarcoma.

To dispense the cryogenic fluid, dispensing heads in accordance with various exemplary embodiments of the present teachings may be used in conjunction with containers defining reservoirs containing a cryogenic fluid. The dispensing heads may provide, for example, an actuation member configured to move between a first position and a second position. When in a first position, the actuation member can block a flow passage outlet opening, thereby preventing the flow of the cryogenic fluid from the reservoir containing the cryogenic fluid and a dispensing member. When moved into a second position, however, the actuation member can align the flow passage outlet opening with a lumen inlet opening of the dispensing member, thereby placing the flow passage outlet opening in flow communication with the lumen inlet opening and allowing flow of the cryogenic fluid from the reservoir to the dispensing member.

To limit and/or regulate the flow rate of the cryogenic fluid, dispensing heads in accordance with various exemplary embodiments of the present teachings may utilize at least one porous member in the flow path of the cryogenic fluid. The at least one porous member may, for example, be configured as a primary flow regulation mechanism to limit the flow rate of the cryogenic fluid flowing from the reservoir through the porous member. As used herein, the term "primary flow regulation mechanism" refers to the main and/or sole mechanism for regulating the flow rate of the cryogenic fluid during dispensing of the cryogenic fluid from the reservoir and out of the device. As used herein, a "primary flow regulation mechanism" regulates the cryogenic fluid flow rate when the device is actuated (i.e., when the device is turned "on") so as to dispense the cryogenic fluid in an amount and at a rate sufficient to safely and effectively effectuate treatment (e.g., without which the flow rate would be too great to effectively use the device for its intended purpose). Thus, as used herein, "primary flow regulation" refers to regulating the cryogenic fluid flow rate once the device is turned on, as opposed to controlling activation of the device (i.e., turning the device "on" and "off" thereby respectively allowing and disallowing fluid flow from the reservoir to be dispensed to a treatment location). In other words, in at least some exemplary embodiments the flow path of the cryogenic fluid may be substantially free of other flow regulation mechanisms, such as, for example, needle valves, orifices, and/or finely cut dispensing tip diameters, other than the at least one porous member and thus flow regulation during actuation and dispensing occurs via the at least one porous member alone. In other exemplary embodiments, if additional flow regulation mechanisms are provided to regulate the flow rate of cryogenic fluid during dispensing of the fluid from a reservoir to a treatment location, such additional flow regulation mechanisms when used with a porous member as a "primary flow regulation mechanism" serve as a secondary regulation of the flow rate of the cryogenic fluid.

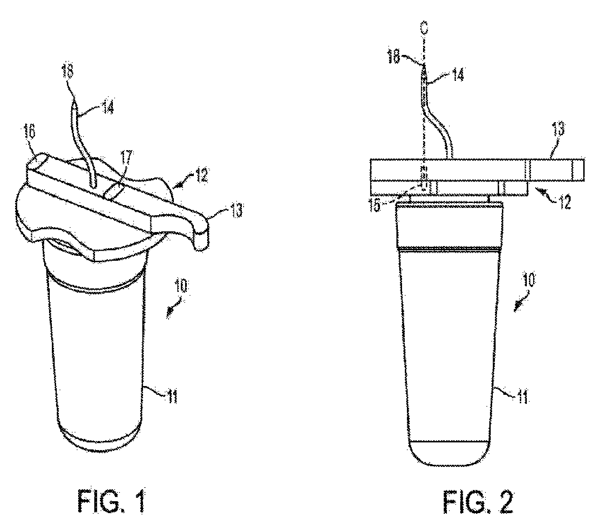

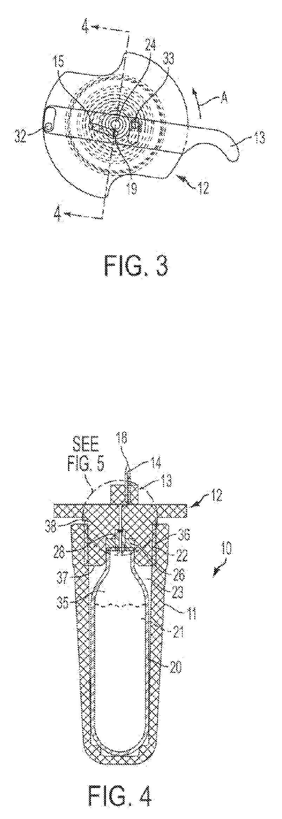

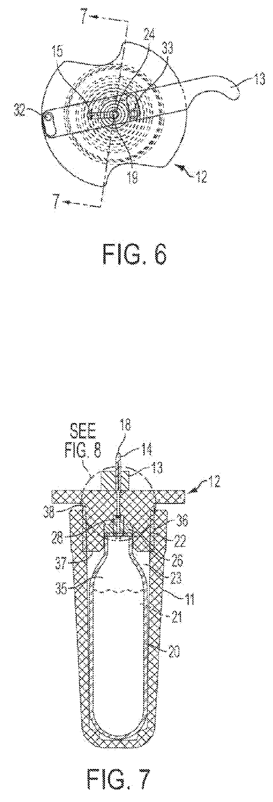

FIGS. 1-8 illustrate an exemplary embodiment of a device for dispensing a cryogenic fluid in accordance with the present teachings. As illustrated in FIGS. 1 and 2, the device 10 for dispensing a cryogenic fluid includes a dispensing head 12, which can engage with a housing 11. As shown in FIGS. 4 and 7, the housing 11 defines a chamber 23 configured to receive a container 20 defining a reservoir 35 for containing a cryogenic fluid 21. The cryogenic fluid 21 may comprise any suitable cryogenic agent fluid and/or mixture of fluids capable of providing reduced temperatures suitable for producing permanent, irreversible rupture of the cellular membranes of a skin lesion tissue, such as, for example, halogenated hydrocarbons (e.g., tetrafluoromethane, trifluoromethane and 1,1,1,2-Tetrafluoroethane), dimethyl ether (DME), n-butane, isobutene, propane, chlorofloro carbons, nitrous oxide and/or liquid nitrogen.

Those skilled in the art would understand that due to the relatively high internal pressures of the container 20, the cryogenic fluid 21 may be in the form of a liquid or a gas/liquid mixture. Those skilled in the art would further understand that the cryogenic fluid 21 may comprise various mixtures of cryogenic substances, which can, for example, permit lower internal pressures of the container 20 to achieve a desired boiling point.

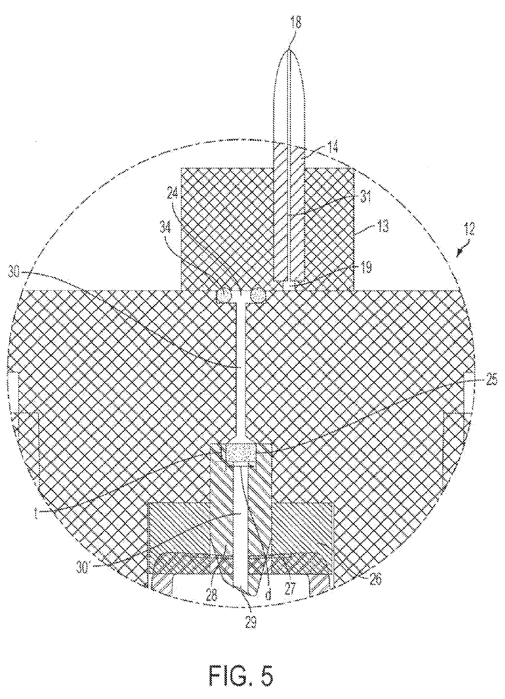

The container 20 is configured to maintain the cryogenic fluid 21 under pressure by a seal 27, as shown in FIG. 5, at least, for example, when the reservoir 35 is at conventional storage, transit, and operating temperatures. The container 20 may be formed from various materials, including, for example, plated steel, aluminum and/or a poly-lined material. The seal 27 may also be formed from various materials, including, for example, aluminum, steel, rubber, plastic and/or a synthetic material. Those ordinarily skilled in the art would understand that the type of material for the container 20 and the seal 27 can be chosen based on resistance to corrosion from contact with the cryogenic fluid 21, ability to withstand the internal pressures and temperatures associated with containing cryogenic fluids, such as, for example, liquid nitrogen, and other such factors.

In various exemplary embodiments of the present teachings the container 20 and other components of the device 10 may be disposable such that the entire device 10 can be thrown away when the cryogenic fluid 21 is used up. In various additional exemplary embodiments, the container 20 may be configured to be recharged with cryogenic fluid upon depletion. In various further exemplary embodiments, the container 20 may be replaced with a new container with cryogenic fluid upon depletion, and remaining components of the device 10 may be reusable.

As illustrated in FIGS. 4 and 7, the dispensing head 12 can be removably engageable with the housing 11 or at least movably engageable with the housing 11. Various exemplary embodiments of the present teachings contemplate, for example, providing the dispensing head 12 and housing 11 with mutually engageable screw threading so as to enable the dispensing head 12 to be moved (e.g. loosened from and tightened onto) the housing 11. In the exemplary embodiment of FIGS. 1-8, as best shown in FIGS. 4 and 7, screw threading 36 may be provided on an external surface of the dispensing head 12 and configured to engage with screw threading 22 on an inner surface of the housing 11. To first use the device 10, an operator may tighten the dispensing head 12 down into the housing 11 via screwing. The dispensing head 12 may define a recess 37 configured to receive at least a portion of a neck 38 of the container 20 when the dispensing head 12 is tightened down onto the housing 11. The dispensing head 12 may further include a hollow piercing member 28 at least partially disposed in the recess 37 and configured to puncture the seal 27, placing the reservoir 35 in flow communication with the dispensing head 12, as will be described in more detail below. Disposed in the recess 37 surrounding the hollow piercing member 28 is a sealing member 26 which may be a compression seal for example, configured to substantially prevent the cryogenic fluid 21 from leaking as it flows from the reservoir 35 through the hollow piercing member 28.

Various exemplary embodiments of the present teachings contemplate various additional piercing mechanisms to puncture seal 27. Those of ordinary skill in the art would understand, therefore, that the hollow piercing member 28 as shown and described herein is exemplary only and not intended to limit the scope of the present teachings. Also, although in the exemplary embodiment of FIGS. 1-8, screw threading is provided on an outer surface of the dispensing head 12 and an inner surface of the housing 11, those ordinarily skilled in the art would appreciate that the dispensing head could have an inner surface portion provided with screw threading configured to engage with screw threading provided on an outer surface of the housing 11.

Various exemplary embodiments of the present teachings (not shown) further contemplate that the dispensing head 12 can engage directly with container 20, which could eliminate the need for the housing 11. For example, to first use the device, an operator may engage screw threading provided on an inner portion of the dispensing head 12 onto screw threading provided on an outer portion of the container 20. As above, when the dispensing head 12 is tightened down onto the container 20, the hollow piercing member 28 can puncture the seal 27, placing the reservoir 35 in flow communication with the dispensing head 12.

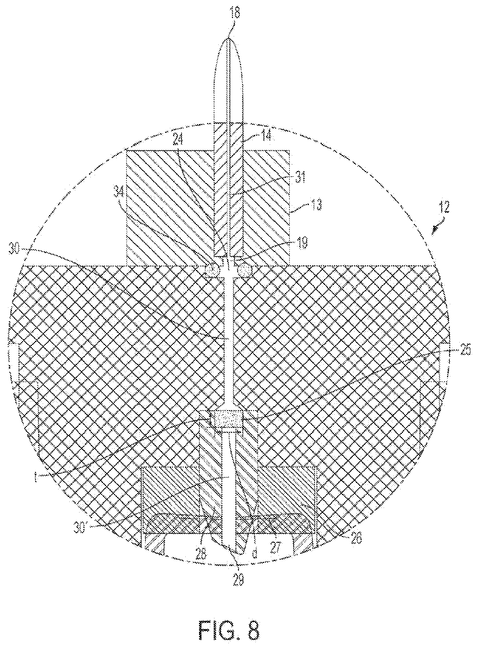

As illustrated in FIGS. 5 and 8, the dispensing head 12 includes a flow passage 30. The flow passage 30 defines, for example, a flow passage net opening 29 and a flow passage outlet opening 24. In various exemplary embodiments, for example, the flow passage 30 may include the hollow portion 30' of the hollow piercing member 28, which may define the flow passage inlet opening 29. Accordingly, when the dispensing head 12 is tightened down onto the housing 11, the hollow piercing member 28 can puncture the seal 27, placing the flow passage net opening 29 of the flow passage 30 in flow communication with the reservoir 35.

Those ordinarily skilled in the art would understand that the material, size and configuration of the flow passage 30 can be chosen based on the type of cryogenic fluid used, resistance to corrosion from contact with the cryogenic fluid, cost, efficiency and other such factors. To allow the cryogenic fluid 21 to travel in an effective and relatively short (Le., direct) path between the flow passage net opening 29 and the flow passage outlet opening 24, various exemplary embodiments of the present teachings contemplate using a substantially straight flow passage 30.

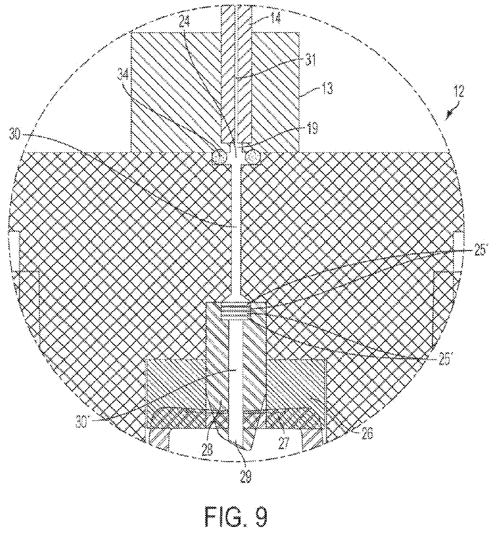

As illustrated by FIGS. 5 and 8, the flow passage 30 includes at least one porous member 25. The at least one porous member 25 can be disposed in the flow passage 30 and configured as a primary flow regulation mechanism to limit a flow rate of the cryogenic fluid 21 that is dispensed from the dispensing head 12. Various exemplary embodiments of the present teachings consider, for example, a substantially disk-shaped porous member 25 having a thickness t ranging from about 0.5 mm to about 12.0 mm, a diameter d ranging from about 1.0 mm to about 5.0 mm, a porosity ranging from about 20% to about 35%, and a pore size ranging from about 0.1 µm to about 170 µm, for example, from about 1.0 µm to about 4.0 µm. Various additional exemplary embodiments of the present teachings consider a plurality (i.e., more than 1) of substantially disk-shaped porous members 25' stacked one on top of the other, as illustrated for example in FIG. 9. In various exemplary embodiments, each of the plurality of porous members 25' may have a thickness ranging from about 0.1 mm to about 3.0 mm and a diameter ranging from about 1.0 mm to about 5.0 mm. In the exemplary embodiment of FIG. 9, the plurality of porous members 25' may each have the same porosity, or at least one of the porous members 25' may have a porosity that differs from the other porous members 25'.

The at least one porous member 25 may be formed from any suitable material and/or combination of materials, including, for example, glass, plastic and/or metal. In various exemplary embodiments, the at least one porous member 25 may be a frit. Various exemplary embodiments in accordance with the present teachings, for example, contemplate that the at least one porous member 25 can be formed from a synthetic fluoropolymer, such as, for example, polytetrafluoroethylene (PTFE). Those ordinarily skilled in the art would understand that the type of material may be chosen based on efficiency, resistance to corrosion from contact with the cryogenic fluid 21, ability to withstand the internal pressures and temperatures that are generated and other factors. Various exemplary embodiments of the present teachings, for example, contemplate that the at least one porous member 25 may withstand a pressure of less than or equal to about 750 psi and a temperature ranging from about 0° C to about -110° C.

Those ordinarily skilled in the art would further understand that the at least one porous member 25 can be cut and/or shaped as desired to properly fit and function within the flow passage 30, and need not be disk-shaped. For example, various exemplary embodiments of the present teachings contemplate that the at least one porous member 25 can be formed within the hollow portion 30' of the hollow piercing member 28.

Various exemplary embodiments of the present teachings consider that the at least one porous member 25 can both limit the flow rate of the cryogenic fluid 21 and filter contaminants out of the cryogenic fluid 21. The at least one porous member 25 may be configured to regulate the rate at which the cryogenic fluid 21 flows from the reservoir 35 to the outlet of the dispensing head 12 (e.g., to outlet 18 of a dispensing member 14) in order to achieve a desired flow rate of the cryogenic fluid 21 for dispensing to a desired location, whether that location is a lesion to be treated or an application member. In the case of cryosurgical applications, it may be desirable to meter the amount of cryogenic fluid that is dispensed during the time period in which the actuation member 13 is in the open position and the device is being used for cryosurgical freezing. Such regulation over the amount of fluid that flows from the dispensing member 18 can help to ensure that sufficient freezing takes place to effect the desired cryosurgical treatment, without over freezing and/or uncontrolled dispensing so as risk contacting locations other than the target location with the cryogenic fluid.

Various exemplary embodiments of the present teachings contemplate, for example, that the at least one porous member 25 is the only mechanism for regulating the flow rate of the cryogenic fluid 21 flowing from the reservoir 35 through the dispensing head 12 to the outlet of the lumen 31, for example, without valves or other similar active flow regulation mechanisms. Various additional exemplary embodiments of the present teachings further consider using various supplemental flow regulation techniques in combination with the at least one porous member 25. For example, in various exemplary embodiments the inner diameter of the flow passage 30 and/or of a lumen 31 in the dispensing member 14 can also help to regulate the flow rate and supplement the use of the at least one porous member 25 as the primary flow regulation mechanism.

As illustrated in FIGS. 1 and 2, the dispensing head 12 further includes an actuation member 13 and a dispensing member 14 carried by the actuation member 13. The actuation member 13 may be affixed to the base portion of the dispensing head 12 that connects to the container 11. Screws 32 and 33 (shown in FIGS. 3 and 6), which are located within screw holes 16 and 17 respectively, may be used to attach the actuation member 13; although such fixation is exemplary and non-limiting and those skilled in the art would understand other manners in which to fix the actuation member 13 to the base portion of the dispensing head 12. The actuation member 13 may move between a first position and a second position. For example, in the exemplary embodiment of FIGS. 1-8, the actuation member 13 may comprise a lever arm that can rotate the actuation member 13 about a center of rotation C between the first position and the second position. Movement of the actuation member 13 in turn can move the dispensing member 14, as will be explained in further detail below.

In various exemplary embodiments, the degree to which the actuation member 13 moves between the first and second positions can be controlled. For example, in the exemplary embodiment of FIGS. 1-8, the size (e.g., length) and configuration of the screw holes 16 and 17 can regulate how much the actuation member 13 can move between the first position and the second position. For example, in various embodiments, the configuration of the movement of the actuation member 13 may be chosen based on the degree of rotation desired for actuation member 13. As illustrated in FIGS. 3 and 6, various exemplary embodiments of the present teachings consider, for example, that screw holes 16 and 17 are sufficiently sized to allow actuation member 13 to rotate in a fixed path, with a fixed degree of rotation. Various exemplary embodiments contemplate, for example, that the actuation member 13 may rotate about 20 degrees between the first position and the second position. Those ordinarily skilled in the art would understand that the type, size and configuration of screws 32 and 33 can be chosen based on the size and configuration of screw holes 16 and 17, cost, efficiency and other such factors.

As illustrated in FIG. 2, in various exemplary embodiments of the present teachings, the actuation member 13 may rotate about a pin 15, which controls the center of rotation C of the actuation member 13 so that the center of rotation C substantially aligns with a lumen outlet opening 18 of the dispensing member 14. Accordingly, the actuation member 13 with the dispensing member 14 can rotate substantially without effecting the position of the lumen outlet opening 18 of the dispensing member 14.

In various exemplary embodiments the actuation member 13 may be biased, such as, for example, to move into a closed position when the actuation member 13 is not held in an open position. In other exemplary embodiments, the actuation member 13 may stay in the first or second position once moved there until a user moves the actuation member 13 back to the other position.

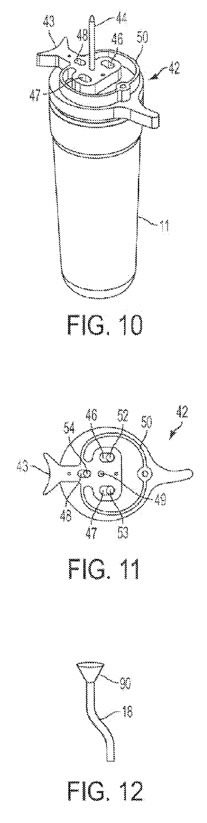

As illustrated, for example, in FIGS. 10 and 11, various additional exemplary embodiments of the present teachings further consider a dispensing head 42 including an actuation member 43 comprising a lever arm that can slide the actuation member 43 between the first position and the second position. Movement of the actuation member 43 in turn can move a dispensing member 44, as will be explained in further detail below.

As before, in various exemplary embodiments, the degree of movement of the actuation member 43 between the first and second position may be controlled based on the configuration of the actuation member 43. For example, the size (e.g., length) and configuration of screw holes 46 and 47 and an alignment pinhole 48 can regulate how much the actuation member 43 moves between the first position and the second position. For example, in various embodiments, the size and configuration of the screw holes 46 and 47 and the alignment pinhole 48 can be chosen based on the degree of slide desired for actuation member 43. As illustrated in FIG. 11, various exemplary embodiments of the present teachings consider, for example, that the screw holes 46 and 47 and the alignment pinhole 48 are sufficiently sized to allow actuation member 43 to slide in a fixed path, with a fixed degree of movement between the first position and the second position.

Those ordinarily skilled in the art would understand that the type, size and configuration of screws 52 and 53 and an alignment pin 54 can be chosen based on the size and configuration of the screw holes 46 and 47 and the alignment pinhole 48, cost, efficiency and other such factors. In various exemplary embodiments, for example, the alignment pinhole 48 and the alignment pin 54 may be replaced, for example, with an additional screw hole and screw.

In various exemplary embodiments of the present teachings, the actuation member 43 may be biased, such as, for example, to move into a closed position when the actuation member 43 is not held in an open position. As illustrated in FIG. 11, for example, the actuation member 43 may comprise a tension spring 50 to slide into a closed position when the actuation member 43 is not held in an open position. In other exemplary embodiments, the actuation member 43 may stay in either the first position or the second position once moved there until a user moves the actuation member 43 back to the other position.

Remaining components of the cryosurgical device of the exemplary embodiment of FIGS. 10 and 11 are the same as those described with reference to the exemplary embodiments of FIGS. 1-8 and are therefore not described in detail herein.

With reference again to FIGS. 3-5, the device 10 is shown with the actuation member 13 of the dispensing head 12 in a first position, wherein flow of the cryogenic fluid 21 out of the flow passage 30 and into the dispensing member 14 is blocked.

As illustrated in FIGS. 4 and 5, the actuation member 13 is positioned so that the flow passage outlet opening 24 is not aligned with and is blocked from flow communication with a lumen inlet opening 19 of the dispensing member 14, thereby preventing flow of the cryogenic fluid 21 from the container 20 to the dispensing member 14. In other words, in this position of the actuation member 14, the device 10 is "off," and can be actuated or tuned "on," for example, by moving the actuation member 13 (e.g., along path A in FIG. 3 or in the exemplary embodiment of FIGS. 10 and 11 moving actuation member 43 toward a center of the device) to a second position.

With reference now to FIGS. 6-8, the device 10 is shown with the actuation member 13 of the dispensing head 12 in a position wherein flow of the cryogenic fluid 21 out of the dispensing member 14 is allowed.

As illustrated in FIGS. 7 and 8, the actuation member 13 is positioned so that the flow passage outlet opening 24 is aligned with and in flow communication with the lumen inlet opening 19, thereby allowing flow of the cryogenic fluid 21 from the container 20 to the dispensing member 14.

The dispensing member 14 defines a lumen 31 having a lumen inlet opening 19 and a lumen outlet opening 18. As described above, and illustrated in FIG. 8, when the actuation member 13 is in the position shown, the lumen inlet opening 19 can be aligned with the flow passage outlet opening 24, creating a flow path between the flow passage inlet opening 29 and the lumen outlet opening 18 to dispense the cryogenic fluid 21. To allow the cryogenic fluid 21 to travel along a path configured to minimize losses and dispensing time, various exemplary embodiments of the present teachings consider that, in the second position, the actuation member 13 can form a substantially straight flow path for the cryogenic fluid 21 between the flow passage inlet opening 29 and the lumen inlet opening 19.

As shown best in FIGS. 5 and 8, the dispensing head 12 may include a sealing member 34, such as, for example, a high durometer o-ring valve, surrounding the flow passage outlet opening 24 and disposed adjacent the actuation member 13. The sealing member 34 may prevent leakage of fluid 21 between the flow passage outlet opening 24 and the actuation member 13. To prevent failure of the sealing member 34 at high pressures, for example, various exemplary embodiments of the present teachings consider a 90 A durometer o-ring. Those ordinarily skilled in the art would understand, however, that various types of sealing mechanisms may be used without departing from the scope of the present teachings.

The dispensing member 14 may be made of any suitable material and/or combination of materials, including, for example, glass, plastic and/or metal. Various exemplary embodiments in accordance with the present teachings contemplate, for example, that the dispensing member 14 can be made of the organic polymer polyaryletheretherketone (PEEK). Those skilled in the art would understand, therefore, that a variety of materials may be chosen for the dispensing member 14 based on cost, efficiency, resistance to corrosion from contact with the cryogenic fluid 21, ability to withstand the temperatures that are generated and other factors.

The dispensing member 14 can dispense the cryogenic fluid 21 via the lumen outlet opening 18. Various exemplary embodiments of the present teachings, for example, contemplate that the dispensing member 14 can dispense the cryogenic fluid 21 at a temperature ranging from about -20° C to about -100° C at the lumen outlet opening 18. In various exemplary embodiments, the dispensing member 14 can dispense the cryogenic fluid 21, for example, directly onto a target freeze area, such as, for example, a skin lesion. In various additional exemplary embodiments, the dispensing member 14 can dispense the cryogenic fluid 21 onto an outer surface portion of an applicator that is separate from the device 10 for subsequent application to the skin lesion via the applicator. Yet further exemplary embodiments contemplate that the dispensing member 14 can dispense the cryogenic fluid 21 to an applicator, for example, into a central portion of an applicator, attached in flow communication with the lumen outlet opening 18. Accordingly, the dispensing member 14, including the lumen outlet opening 18, can have various sizes, shapes and/or configurations based upon a desired application and/or treatment. Those ordinarily skilled in the art would understand, therefore, that the dispensing member 14, as depicted in FIGS. 1-8, is exemplary only, and a variety of dispensing mechanisms may be utilized to dispense cryogenic fluid out of the container 20.

For direct dispensing applications, for example, an inner diameter of the lumen outlet opening 18 can control the spray and/or jet pattern of the cryogenic fluid 21. For this purpose, various exemplary embodiments of the present teaching consider the lumen outlet opening 18 having an inner diameter ranging from about 0.003 inches to about 0.030 inches. Various exemplary embodiments of the present teachings further consider a variety of hollow fluid retaining and/or constricting devices, such as, for example, a contact cone or receiver, which can be used in conjunction with the device 10, to pool the cryogenic fluid and limit the spread of the freeze to a directed location. Disposable fluid retaining devices 90, such as, for example, neoprene cones or commonly available otoscopic cones, can attach directly to the lumen outlet opening 18, as depicted in FIG. 12, and be removed after each use or treatment.

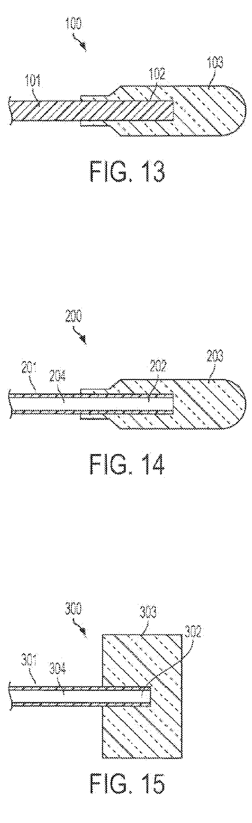

As mentioned above, the dispensing member 14 can also dispense (e.g., spray and/or drip) the cryogenic fluid 21 onto an applicator separate from the device 10 for subsequent application to a skin lesion via the applicator. FIG. 13 illustrates an exemplary applicator 100 that can be used in conjunction with the device 10. The applicator 100 may include, for example, a gripping portion, such as a stem 101, and an application member, such as for example a bud-shaped tip 103, disposed on and secured to an end 102 of the stem 101. The dispensing member 14 can dispense the cryogenic fluid 21 directly onto an outer surface of the bud-shaped tip 103, where the cryogenic fluid 21 may accumulate on the bud-shaped tip 103 and may evaporate therefrom to cool the bud-shaped tip 103. The bud-shaped tip 103 can then be placed in contact with the skin surface of the lesion to cryosurgically treat the lesion.

In an alternative exemplary embodiment, the dispensing member 14 can dispense the cryogenic fluid 21 to an applicator in flow communication with the lumen outlet opening 18. FIG. 14 illustrates an exemplary embodiment of an applicator 200 that can be attached to the device 10. The applicator 200 may include, for example, a hollow tube 201, defining a passage 204, in flow communication with the lumen outlet opening 18 of the dispensing member 14. The hollow tube 201 includes an open discharge end 202, which may be surrounded by an application member, such as for example a bud-shaped tip 203. Alternatively, the bud-shaped tip 203 may be inserted directly over the dispensing member 14, without the intervening tube 201. When the device 10 is actuated, for example, the cryogenic fluid 21 can flow from the lumen outlet opening 18 into the passage 204 of the hollow tube 201, where the cryogenic fluid 21 may accumulate inside the bud-shaped tip 203 and evaporate, thereby cooling the bud-shaped tip 203 via heat of vaporization principles. The bud-shaped tip 203 can then be placed in contact with the skin surface of the lesion to cryosurgically treat the lesion in accordance with the present teachings. In various exemplary embodiments, for example, the bud-shaped tip 203 and tube 201 may be removed from the dispensing member 14 once filled and then the bud-shaped tip 203 may be placed in contact with the target freeze area.

FIG. 15 illustrates an additional exemplary embodiment of an applicator 300 that can be attached to the device 10. The applicator 300 may include, for example, a hollow tube 301, defining a passage 304, in flow communication with the lumen outlet opening 18 of the dispensing member 14. The hollow tube 301 includes an open discharge end 302, which may be surrounded by an application member, such as for example a disk-shaped tip 303. Alternatively, the disk-shaped tip 303 may be inserted directly over the dispensing member 14, without the intervening tube 301. In various exemplary embodiments of the present teachings, the disk-shaped tip 303 can be placed in contact with a skin surface of a lesion. When the device 10 is actuated, for example, the cryogenic fluid 21 can flow from the lumen outlet opening 18 into the passage 304 of the hollow tube 301 (or directly into a central portion of the disk-shaped tip 303), where the cryogenic fluid 21 may accumulate inside the disk-shaped tip 303 and diffuse to the skin surface of the lesion at a temperature that is directly effective to freeze the skin surface such that permanent, irreversible rupture of cellular membranes of cells of the skin surface occurs while the cryogenic fluid is being delivered to the skin surface.

Application members in accordance with various exemplary embodiments of the present teachings, such as, for example, bud-shaped tips 103 and 203 and disk-shaped tip 303, can be made of any suitable porous and/or absorbent material, such as, for example, porex(R), cotton wool, open-celled foams, a sintered thermoplastic, a sintered metal, a glass or ceramic frit, or a polyolefin or polyester non-woven fabric. Those ordinarily skilled in the art would further understand that the above disclosed bud-shaped tips 103 and 203 and disk-shaped tip 303 are exemplary only and that application members in accordance with the present teachings can have various shapes and sizes without departing from the scope of the present teachings. Those ordinarily skilled in the art would understand, for example, that the material, size, shape and/or configuration of the applicator used can be chosen based upon the desired treatment and/or temperature requirements. For example, in various exemplary embodiments, the dispensing member 14 can dispense the cryogenic fluid 21 to an application member configured to cool through heat of vaporization.

In accordance with various exemplary embodiments of the present teachings, an exemplary method for using the device 10 of the exemplary embodiment of FIGS. 1-8 will now be described. To dispense a sufficient and substantially uniform amount of a cryogenic fluid 21 to cryosurgically treat a lesion, such as for example, a skin lesion, an operator may, for example, couple the dispensing head 12 to the housing 11 holding the sealed container 20 filled with a cryogenic fluid 21. The dispensing head 12, for example, may be tightened down onto the housing 11 via engagement of screw threading 22 with screw threading 36. As the dispensing head 12 is tightened down relative to the housing 11, the hollow piercing member 28 may breach the seal 27 on the container 20, placing the flow passage 30 of the dispensing head 12 in flow communication with the reservoir 35 containing the cryogenic fluid 21.

The operator may then invert the device 10 such that the cryogenic liquid in the reservoir 35 is closer to the flow passage inlet opening 29 than is a gas in the reservoir 35. The operator may actuate the dispensing head 12, for example, by rotating the actuation member 13 in the direction of arrow A in FIG. 3 from a first position (shown in FIGS. 3-5), in which an outlet 24 of the flow passage 30 is not aligned and is blocked from flow communication with a dispensing member inlet opening 19, to a second position (shown in FIGS. 6-8), in which the flow passage outlet opening 24 is aligned with and in flow communication with the dispensing member inlet opening 19. Upon actuation, an amount of cryogenic fluid 21 may flow from the reservoir 35 toward the dispensing member inlet opening 19 through the flow passage 30, wherein the flow rate of the cryogenic fluid is primarily regulated by passing the cryogenic fluid 21 through at least one porous member 25 in the flow passage 30. The operator may then dispense the cryogenic fluid from the dispensing member 14 to a target location.

In various additional exemplary embodiments, the operator may actuate the dispensing head 42, for example, by sliding the actuation member 43 toward a center of the device from a first position, in which an outlet 24 of the flow passage 30 is not aligned and is blocked from flow communication with a dispensing member inlet opening 49, to a second position, in which the flow passage outlet opening 24 is aligned with and in flow communication with the dispensing member inlet opening 49. Upon actuation, an amount of cryogenic fluid 21 may flow from the reservoir 35 toward the dispensing member inlet opening 49 through the flow passage 30, wherein the flow rate of the cryogenic fluid is primarily regulated by passing the cryogenic fluid 21 through at least one porous member 25 in the flow passage 30. The operator may then dispense the cryogenic fluid from the dispensing member 44 to a target location.

Various exemplary embodiments of the present teachings contemplate, for example, dispensing the cryogenic fluid 21 directly onto a surface to be treated from the dispensing outlet 18, or flowing or dispensing the cryogenic fluid to an applicator first for subsequent application to the surface to be treated.

For example, in accordance with various exemplary embodiments of the present teachings, an exemplary method for dispensing a cryogenic fluid via an applicator of the exemplary embodiment of FIG. 15 will now be described. To dispense a sufficient and substantially uniform amount of a cryogenic fluid 21 to cryosurgically treat a lesion, such as for example, a skin lesion, an operator may place an application member, such as, for example disk-shaped tip 303, in contact with a skin surface, and supply the cryogenic fluid 21 to the application member while the application member is in contact with the skin surface. The application member may be adapted to diffuse the cryogenic fluid to the skin surface at a temperature that is directly effective to freeze the skin surface such that permanent, irreversible rupture of cellular membranes of cells of the skin surface occurs while the cryogenic fluid is being delivered to the skin surface. For example, the application member may comprise a porex(R) material or other similar sintered thermoplastic or metal that may diffuse the cryogenic fluid to the skin surface. In an exemplary embodiment wherein the cryogenic fluid is liquid nitrogen or nitrous oxide, the cryogenic fluid may be delivered to the skin's surface at a temperature ranging from about -90° C. to about -110° C.

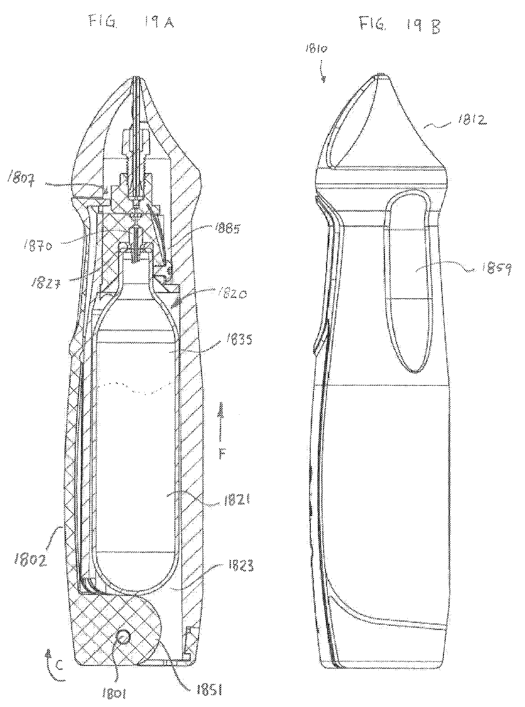

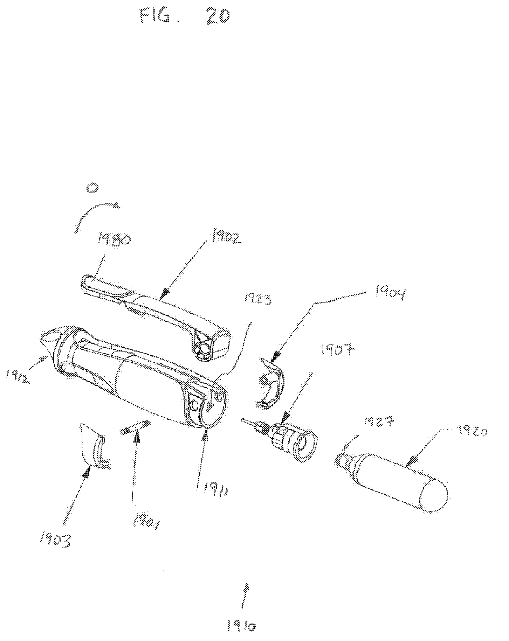

FIGS. 19-22 illustrate one example of a device for dispensing a cryogenic fluid in accordance with the claimed invention. As illustrated in FIGS. 19A-B and in FIG. 20, the device 1910 for dispensing a cryogenic fluid includes a dispensing head 1912 and housing 1911. Housing 1911 includes an activation lever 1902 that can be engaged with a rear-portion of housing 1911 using a connecting pin 1901. Rear caps 1903, 1904 can be attached to housing 1911 and used to cover connecting pin 1901 while permitting lever action of activation lever 1902. Housing 1911 of device 1910 is configured to receive a valve assembly 1907 as well as a container 1920 sealed with seal 1927 and containing a cryogenic fluid (shown as reference numeral 1821 in FIG. 19A and described above).

As also shown in FIGS. 19A and 20, the container 1920 is configured to maintain the cryogenic fluid (shown as reference numeral 1821 in FIG. 19A) under pressure by a seal 1927, for example, when the cryogen reservoir (reference numeral 1835 in FIG. 19A) is at conventional storage, transit, and operating temperatures.

As above, the container 1920 and other components of the device 1910 can be disposable such that the entire device 1910 can be thrown away when the cryogenic fluid 1821 is used up. In additional examples of the claimed invention, the container 1920 can be configured to be recharged with cryogenic fluid upon depletion. Likewise, the container 1920 can be replaced with a new container with cryogenic fluid upon depletion, and remaining components of the device 1910 can be reused or thrown away.

As shown in the exploded view of the device 1910 illustrated in FIG. 20, the dispensing head 1912 can be engageable with the housing 1911. As described above, for example, the dispensing head 1912 and housing 1911 can be provided with mutually engageable screw threads to enable the dispensing head 1912 to be moved (e.g. loosened from and tightened) onto the housing 1911. Likewise, the valve assembly 1907 can be engaged in the dispensing head 1912 and housing 1911 prior to use.

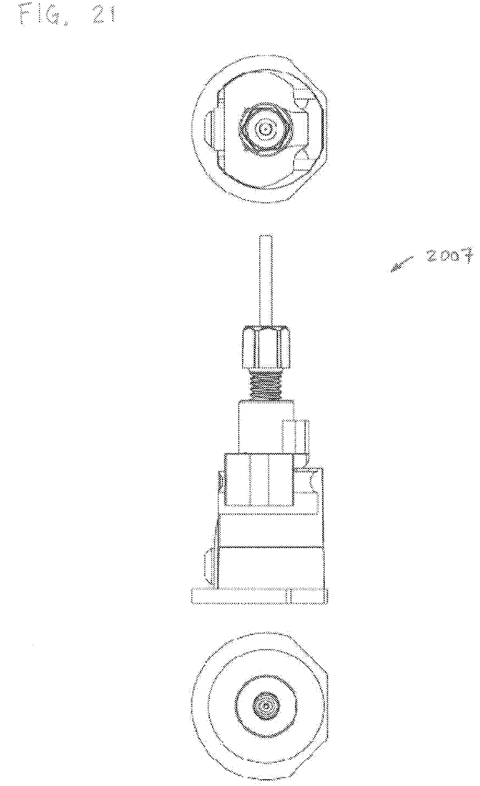

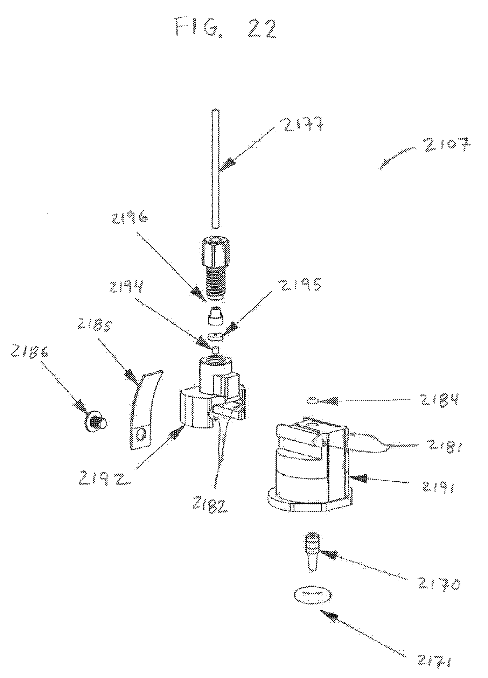

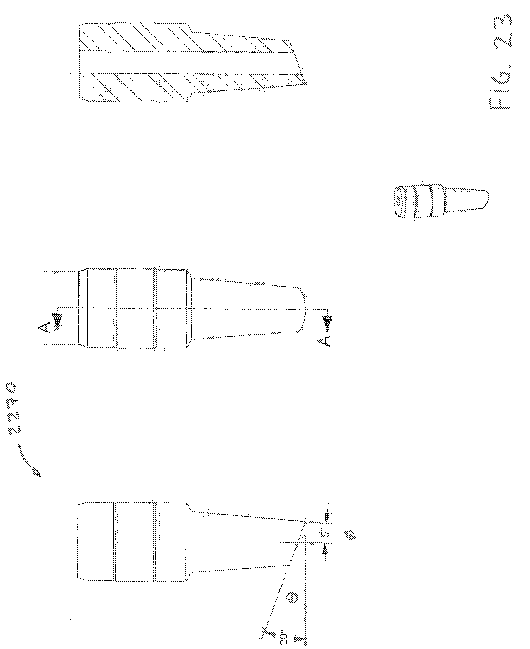

FIG. 21 shows an assembled valve assembly 2007, while FIG. 22 illustrates an exploded view of valve assembly 2107. As shown further in FIGS. 20 and 22, valve assembly 2107 can include valve body 2191 that includes a hollow pierce pin 2170 and o-ring 2171 that extend downward toward the rear portion of housing 1911 (shown in FIG. 20) when the valve assembly 1907 is engaged with housing 1911 and dispensing head 1912. The pierce pin 1970 is partially disposed in the recess of housing 1911 and can be used to puncture seal 1927 of container 1920 to allow the reservoir of cryogenic fluid in container 1920 to be placed in flow communication with valve assembly 1907 and to allow flow from the container 1920 toward the valve assembly 1907. Pierce pin 2170 can be a right angle design, or the end of pierce pin 2170 can be angled or beveled from the horizontal or vertical axes, for example from 1 to 30 degrees, to puncture the seal 1927 of container 1920 as well. An example of this angling and beveling of the pierce pin is shown in FIG. 23, where from pierce pin 2270 includes a vertical angle Φ of 5 degrees and a beveled angle θ of 20 degrees.

Returning to FIG. 22, o-ring 2171 provides a positive seal between the pierce pin 2170 and container 1920 and is configured to substantially prevent the cryogenic fluid (shown as reference numeral 1821 in FIG. 19) from leaking as it flows between the reservoir of container 1920 to the pierce pin 2170 of the valve assembly 2107.

Valve assembly 2107 also includes a valve slide 2192 machined to slide over and engage the valve body 2191 when the valve assembly 2107 is engaged with housing 1911 and dispensing head 1912 of the device 1910. Valve slide 2192 can incorporate a number of different geometries to provide a slidably engageable fit over valve body 2191. In one example of the claimed invention shown in FIG. 22, valve slide 2192 includes recessed keyways 2182 machined to receive keys 2181 machined on valve body 2191. Of course other key geometries can be used to slidably engage the valve slide 2192 over valve body 2191. O-ring 2184 is installed between the valve body 2191 and the valve slide 2192 to ensure a positive seal between the valve body 2191 and the valve slide 2192 as they are engaged. O-ring 2184 surrounds the respective openings on valve body 2191 and valve slide 2192 that align to form a conduit for the delivery of cryogenic liquid. O-ring 2184 is configured to substantially prevent cryogenic fluid 1821 from uncontrollably leaking as it flows between valve body 2191 and valve slide 2192. This serves to protect both the user of the device and the patient being treated.

Additionally, the o-ring 2184 sealing member surrounding the flow passage outlet opening that is disposed adjacent to the valve slide can be made using synthetic rubber including Buna rubber, nitrile rubber, styrene-butadiene rubber, nitrile butadiene rubber, silicone, and the like. These materials provide a positive seal at the lower operating temperatures of about -90° C to about -110° C and are not as susceptible to freezing or losing their mechanical seal than other materials. Other materials such as silicone and natural rubbers can also be used.

As shown in FIGS. 5, 8, and 22, valve assembly 2107 further includes a valve spring 2185, a valve spring screw 2186, a filter 2194, a backer disk 2195, a compression fitting 2196, and extension tube 2177. The filter 2194 and backer disk 2195 can be made from a variety of materials depending upon the type, amount, and target of the cryogenic fluid. For example, filter 2194 and backer disk 2195 can include high solid filtration and separation materials, including polyethylene substrates, polyvinylidene difluoride (PVDF) membranes, sintered thermoplastic or metals, and the like. The position of the filter 2194 (shown as filter 25 in FIG. 5) downstream of the cryogenic gas eliminates potential valve freeze that can occur. For example, by configuring the filter 2194 between the flow passage inlet opening 29 of the valve block and the flow passage outlet opening 24 of the valve block prevents frozen crystals from forming at the outlet of the valve assembly (that is, the point where the flow passage outlet opening 24 meets the lumen inlet opening 19 of the valve slide.

Returning to FIG. 20, with the housing 1911 and dispensing head 1912 engaged and valve assembly 1907 secured, a user can access a recess 1823 (shown as an open area in FIG. 19A) in housing 1911 by lifting release point 1980 on activation lever 1902. Lifting release point 1980 on activation lever 1902 causes activation lever 1902 to pivot about connecting pin 1901 away from dispensing head 1912 as shown by directional force arrow O. As activation lever 1902 is rotated from a closed to a fully open position, recess 1923 in housing 1911 is accessed. Recess 1923 is configured to receive container 1920 and to securely hold the container 1920 in the recess 1923 of the device 1910.

Referring again to FIG. 19, after container 1820 is inserted into recess 1823, activation lever 1802 is rotated toward dispensing head 1812 to a fully closed position (shown as rotation direction force arrow C in FIG. 19A). As activation lever 1802 is rotated to its fully closed position along C about connecting pin 1801, eccentric hub 1851 contacts a rear portion of container 1820 and applies a cam action upon container 1820, which moves container 1820 transversely in the direction of force arrow F shown in FIG. 19A. As activation lever 1802 is rotated to a fully closed position, pierce pin 1870 pierces seal 1827 of container 1820 which allows the cryogenic fluid 1821 to flow from the container 1820 toward the valve body (shown in FIG. 22 as reference numeral 2191). The valve assembly 1807 can then be used to effect the delivery of the cryogenic fluid 1821 to a target lesion.

Once the container 1820 is inserted in recess 1823 and activation lever 1802 is fully closed, a user can initiate the delivery of the cryogenic fluid 1821 to the patient. To effect delivery of the cryogenic fluid 1821 to the patient, a user actuates the valve assembly 1807. In the example of the claimed invention shown in FIGS. 19-22, a user can press button 1859 that covers valve spring 1885. Valve spring 1885 (also shown as reference numeral 2185 in FIG. 22) slides valve slide 2192 into cooperation with valve body 2191 such that an orifice in valve slide 2192 aligns with an orifice in valve body 2191 thereby allowing cryogenic fluid 1821 to flow from container 1820 through pierce pin 2170 through the orifice in valve body 2191 through the orifice in valve slide 2192 through a porous member, such as filter 2194, through a second porous member such as backer disk 2195, through compression fitting 2196 into extension tube 2177 to an affected area on the patient.

In one embodiment of the claimed invention, the degree to which the button 1859 moves between a first and a second position can be controlled. Likewise, the degree of movement of the valve spring 1885 is affected by the degree of movement of the button 1859, thereby affecting the amount of cryogenic material flows through flow lumen inlet opening 19 (shown in FIGS. 5 and 8) by moving the valve slide into or out of alignment with the flow passage 30 of the valve body.

As illustrated above with regard to FIGS. 5 and 8, the porous members 2194, 2195 shown in FIG. 22 illustrating this example of the claimed invention can be disposed in the flow passage and configured as flow regulation mechanisms to limit a flow rate of the cryogenic fluid 1821 that is dispensed from the dispensing head 1812 and used to treat the patient. Filter 2194 and backer disk 2195 can be porex(R) or other similar sintered thermoplastic or metal members, for example. As above, exemplary embodiments of the claimed invention can use, for example, substantially disk-shaped porous members 2194, 2195 having a thickness t ranging from about 0.1 mm to about 12.0 mm, a diameter d ranging from about 1.0 mm to about 5.0 mm, a porosity ranging from about 10% to about 70%, and a pore size ranging from about 0.1 µm to about 170 µm, for example, and from about 1.0 µm to about 4.0 µm. As was the case illustrated in FIG. 9, and as shown in the illustrated example of FIGS. 19-22, the porous members 2194, 2195 can be stacked one on top of the other, can be the same geometry as each other, can be the same porosity as each other, or alternatively can be selected based upon different geometries and/or different porosities.

Likewise, the porous members 2194, 2195 can be selected based on efficiency, resistance to corrosion from contact with the cryogenic fluid 1821, ability to withstand the internal pressures and temperatures that are generated and other factors. For example, the porous member 2194, 2195 in the example of the claimed invention illustrated in FIGS. 19-22 can withstand a pressure of about 750-2250 psi and a temperature ranging from about 0° C to about -190° C.

The porous members 2194, 2195 can be formed from any suitable material and/or combination of materials, including, for example, glass, plastic, metal, synthetic fluoropolymers, such as, polytetrafluoroethylene (PTFE), and the like. For example, one or more of the porous members 2194, 2195 can be a frit.

As above, the porous members 2194, 2195 can both limit the flow rate of the cryogenic fluid 1821 and filter contaminants out of the cryogenic fluid 1821. The porous members 2194, 2195 can be configured to regulate the rate at which the cryogenic fluid 1821 flows from the reservoir 1835 to the outlet of the dispensing head 1812 to achieve a desired flow rate of the cryogenic fluid 1821 for dispensing to a desired location, whether that location is a lesion to be treated or an application member.

### EXAMPLES

As discussed above, the present teachings contemplate devices that are capable of dispensing a regulated flow of a cryogenic fluid sufficiently cold enough for effective cryosurgical treatment, without over-freezing and/or undesired contact of the cryogenic fluid with other surfaces, such as, for example, healthy tissue or skin surrounding a lesion. To verify the flow regulation capabilities of devices and methods in accordance with exemplary embodiments of the present teachings, various laboratory tests were conducted with and without a porous member disposed in the flow passage. A dispensing head in accordance with the present teachings (i.e., with at least one porous member disposed in the flow passage), for example, demonstrated a flow rate of about 0.035 grams/second, whereas a dispensing head with an unobstructed flow passage (i.e., without a porous member disposed in the flow passage) demonstrated a flow rate of about 0.248 grams/second. Accordingly, given an 8 gram container of cryogenic fluid, a device which utilized the dispensing head without at least one porous member depleted its supply of cryogenic fluid much faster than a device which utilized the dispensing head with at least one porous member (e.g., 32 seconds vs. 228 seconds). Assuming a treatment time of 3 seconds per application, the device which utilized the dispensing head without at least one porous member could, therefore, only perform approximately 10 treatments, whereas the device which utilized the dispensing head with at least one porous member could perform approximately 76 treatments. The tests, therefore, demonstrated that the devices and methods in accordance with exemplary embodiments of the present teachings can dispense a regulated flow of a cryogenic fluid in order to achieve a desired treatment regime.

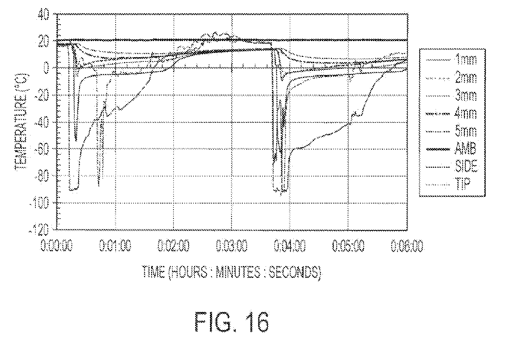

To further verify the cooling efficiency of devices and methods in accordance with exemplary embodiments of the present teachings, various additional laboratory tests were conducted with the results being illustrated in FIGS. 16-18.

In FIG. 16, for example, a device having a configuration similar to that of FIGS. 1-8 in accordance with the present teachings was used to dispense nitrous oxide (N₂O) directly onto a target freeze area on a gelatin material which was used to simulate skin lesion tissue. In the experiment, two open sprays of N₂O were applied directly to the gelatin surface and thermocouples were used to measure the ambient temperature (i.e., AMB in FIG. 16), temperatures t the lumen outlet opening 18 (i.e., TIP in FIG. 16) and the area adjacent to the lumen outlet opening (i.e., SIDE in FIG. 16), and at different depths in the gelatin (i.e., 1 mm, 2 mm, 3 mm, 4 mm and 5 mm in FIG. 16). FIG. 16 is a plot of the measured temperatures over a six minute period.

As illustrated by the TIP thermocouple data in FIG. 16, the device was able to dispense N₂O at temperatures as low as about -90° C. This resulted in an almost immediate freezing of the target area to about -56° C, as demonstrated by the 1 mm thermocouple reading.

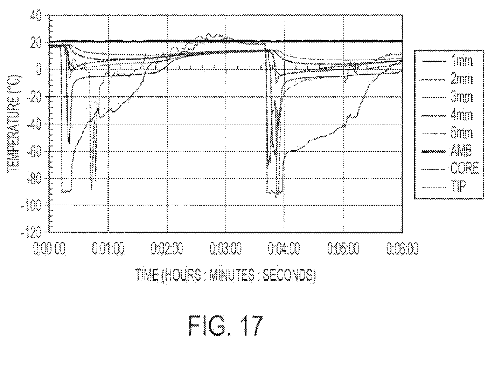

In FIG. 17, a device having a configuration similar to that of the exemplary embodiment of FIGS. 1-8 in accordance with the present teachings was used in conjunction with a porous polyethylene (50 µm pores) application member to apply nitrous oxide (N₂O) onto the target freeze area of a gelatin material used to simulate skin lesion tissue. In the experiment, NO was dispensed into the application member (i.e., in flow communication with the lumen outlet opening of the device similar to the applicator shown in FIG. 15) in two, ten second intervals while the application member was in contact with the gelatin surface and thermocouples were used to measure ambient temperature (i.e., AMB in FIG. 17), temperatures at the disk-shaped tip (i.e., TIP in FIG. 17) and the applicator core (i.e., CORE in FIG. 17), and at different depths in the gelatin (i.e. 1 mm, 2 mm, 3 mm, 4 mm and 5 mm in FIG. 17). FIG. 17 is a plot of the measured temperatures over a six minute period.

As illustrated by the TIP thermocouple trace in FIG. 17, the device was again able to dispense N₂O via the applicator at temperatures as low as about -90° C. This resulted in an almost immediate freezing of the target area to about -56° C, as demonstrated by the 1 mm thermocouple reading.

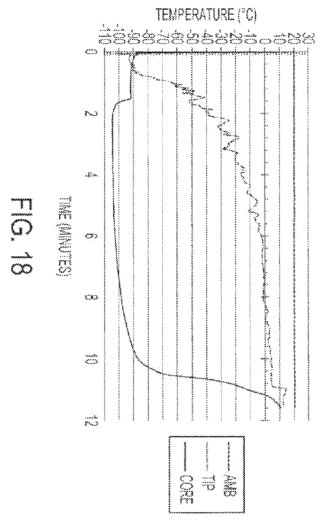

In FIG. 18, a device having a configuration similar to that of the exemplary embodiment of FIGS. 1-8 in accordance with the present teachings was used in conjunction with an open-celled foam application member to demonstrate heat of vaporization cooling. In the experiment, N₂O was dispensed into the application member (i.e., in flow communication with the lumen outlet opening of the device similar to the applicator shown in FIG. 14) and the application member was allowed to cool for approximately 30 seconds (i.e., to allow for heat of vaporization cooling) prior to contact with the gelatin surface. Thermocouples were used to measure ambient temperature (i.e., AMB in FIG. 18), and temperatures at the bud-shaped tip (i.e., TIP in FIG. 18) and the applicator core (i.e., CORE in FIG. 18), FIG. 18 is a plot of the measured temperatures over a twelve minute period.

As illustrated by the TIP thermocouple trace in FIG. 18, the application member exhibited an almost immediate drop in temperature to about -100° C. as the N₂O evaporated from the applicator (i.e., through heat of vaporization).

Accordingly, FIGS. 16-18 demonstrate that the devices and methods in accordance with exemplary embodiments of the present teachings can dispense cryogenic fluids so as to achieve cooling of application members as low as about 100° C.

For the purposes of this specification and appended claims, unless otherwise indicated, all numbers expressing quantities, percentages or proportions, and other numerical values used in the specification and claims, are to be understood as being modified in all instances by the term "about" if they are not already. Accordingly, unless indicated to the contrary, the numerical parameters set forth in the following specification and attached claims are approximations that may vary depending upon the desired properties sought to be obtained by the present teachings. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the present teachings are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all sub-ranges subsumed therein.

It is noted that, as used in this specification and the appended claims, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

It should be understood that while the invention has been described in detail with respect to various exemplary embodiments thereof, it should not be considered limited to such, as numerous modifications are possible without departing from the broad scope of the appended claims.

## Claims

1. An activation valve assembly (2107) comprising:
a valve body (2191) including a flow passage (30) with a flow passage inlet opening (29) that receives a cryogenic material (1821) and a flow passage outlet opening (24) that delivers the received cryogenic material (1821); and
a valve slide (2192) slidably engaged with the valve body (2191), the valve slide (2192) including a lumen inlet opening (19) for receiving cryogenic material (1821) from the flow passage outlet opening (24) and a lumen outlet opening (18) that delivers the cryogenic material (1821) to a target;
wherein the valve slide (2192) is engaged with the valve body (2191) and positioned in a closed position where the lumen inlet opening (19) is offset from the flow passage outlet opening (24) of the valve body (2191) to prevent flow of the cryogenic material (1821);
wherein the valve slide (2192) is engaged with the valve body (2191) and slidably positioned in an open position where the lumen inlet opening (19) is aligned with the flow passage outlet opening (24) of the valve body (2191) to form a flow path to allow flow of the cryogenic material (1821) through the valve assembly; and
wherein the valve slide (2192) includes recessed keyways (2182) machined to receive keys (2181) machined on the valve body (2191).

2. The activation valve assembly (2107) of claim 1, further comprising:
a sealing member (2184) surrounding the flow passage outlet opening (24) and disposed adjacent the valve slide (2192),
wherein the sealing member (2184) preferably comprises synthetic rubber including at least one of Buns rubber, nitrile rubber, styrene-butadiene rubber, or nitrile butadiene rubber.

3. The activation valve assembly (2107) of claim 1, further comprising:
a dispensing member (1812) disposed in the valve slide (2192) and including the lumen inlet opening (19) and the lumen outlet opening (18),
wherein the dispensing member (1812) preferably comprises polyaryletheretherketone (PEEK).

4. The activation valve assembly (2107) of claim 1, wherein the target includes at least one of a target lesion, an applicator or a fluid-retaining device, or wherein the flow of the cryogenic material (1821) through the valve assembly (2107) is at a rate of about 0.035 g/sec.

5. The activation valve assembly (2107) of claim 1, wherein the flow passage inlet opening (29) includes a piercing member (2170), wherein the piercing member preferably includes at least one of a vertical angle from 1-10 degrees and a beveling angle of from 5 to 30 degrees.

6. The activation valve assembly (2107) of claim 1, further comprising:
a filter (2194) disposed in the flow passage (30) between the flow passage inlet opening (29) and the flow passage outlet opening (24),
wherein the filter (2194) preferably comprises a porous material configured to withstand a pressure up to about 750-2250 psi and a temperature ranging from about 0° C to about -190°C, or
wherein the filter (2194) preferably has a thickness ranging from about 0.5 mm to about 12.0 mm, or
wherein the filter (2194) preferably has a porosity ranging from about 20% to about 35%, or
wherein the filter (2194) has a pore size ranging from about 1 µm to about 4 µm, or
wherein the filter (2194) preferably is substantially disk-shaped and has a diameter ranging from about 1.0 mm to about 5.0 mm.

7. The activation valve assembly (2107) of claim 1, further comprising:
a sealing member (2171) surrounding the flow passage inlet opening (29), wherein the sealing member (2171) preferably comprises synthetic rubber including at least one of Buna rubber, nitrile rubber, styrene-butadiene rubber, or nitrile butadiene rubber.

8. A cryogenic fluid dispensing device (1910) comprising:
an activation valve assembly (2107) according to claim 1;
the valve body (2191) being configured to be placed in flow communication with a reservoir (1835) containing a cryogenic fluid, the flow passage inlet opening (29) being configured to receive cryogenic material (1821) from the reservoir (1835); and
an actuation member (13) configured to move between a first position in which the valve slide (2192) is engaged with the valve body (2191) and positioned in a closed position where the lumen inlet opening (19) is not aligned and is blocked from flow communication with the flow passage outlet opening (24) of the valve body (2191) and a second position in which the valve slide (2192) is engaged with the valve body (2191) and slidably aligned in an open position where the lumen inlet opening (19) is aligned with the flow passage outlet opening (24) of the valve body (2191) to form a flow path (29, 30, 31) to allow flow of the cryogenic material (1821) through the valve assembly (2107).

9. The cryogenic fluid dispensing device (1910) of claim 8, wherein
the device (1910) is configured so that when in use for dispensing, a cryogenic liquid in the reservoir (1835) is closer to the flow passage inlet opening (29) than a gas in the reservoir (1835), or
wherein the valve body (2191) further comprises a hollow piercing member (2170) configured to puncture a container (1820) housing the cryogenic material (1821) and to place the valve body (2191) in flow communication with the reservoir (1835) of cryogenic material (1821), or
wherein the device (1910) of claim 8 further comprises:
an activation lever (1802) disposed in a device housing (1911) and configured to engage the cryogenic material container (1820) to contact the hollow piercing member (2170) to puncture the container (1820) to place the valve body (2191) in flow communication with the reservoir (1835) of cryogenic material (1821), or
wherein the device (1910) of claim 8 further comprises:
at least one filter (2194) disposed in the flow passage (30), the at least one filter (2194) configured as a flow regulation mechanism to limit a flow rate of cryogenic fluid as the cryogenic fluid (1821) flows from the reservoir (1820) to the lumen outlet opening (18).

## Patentansprüche

1. Aktivierungsventilanordnung (2107), aufweisend:
einen Ventilkörper (2191), welcher einen Flussdurchgang (30) mit einer ein kryogenes Material (1821) aufnehmenden Flussdurchgangseinlassöffnung (29) und einer das aufgenommene kryogene Material (1821) ausgebenden Flussdurchgangsauslassöffnung (24) beinhaltet; und
ein Schieberventil (2192), welches mit dem Ventilkörper (2191) verschiebbar in Eingriff steht, wobei das Schieberventil (2192) eine Lumeneinlassöffnung zum Aufnehmen von kryogenem Material (1821) von der Flussdurchgangsauslassöffnung (24) und eine Lumenauslassöffnung (18) zum Ausgeben des kryogenen Materials (1821) an ein Target beinhaltet;
wobei das Schieberventil (2192) mit dem Ventilkörper (2191) in Eingriff steht und in einer geschlossenen Position positioniert ist, bei welcher die Lumeneinlassöffnung (19) von der Flussdurchgangsauslassöffnung (24) des Ventilkörpers (2191) beabstandet ist, um einen Fluss des kryogenen Materials (1821) zu verhindern;
wobei das Schieberventil (2192) mit dem Ventilkörper (2191) in Eingriff steht und verschieblich in einer geöffneten Position positioniert ist, bei welcher die Lumeneinlassöffnung (19) mit der Flussdurchgangsauslassöffnung (24) des Ventilkörpers (2191) fluchtet, um einen Flusspfad zu bilden, um einen Fluss des kryogenen Materials durch die Ventilanordnung zu ermöglichen; und
wobei das Schieberventil (2192) gefertigte vertiefte Keilnute (2182) beinhaltet, welche gefertigt sind, um an dem Ventilkörper (2191) gefertigte Keile (2181) aufzunehmen.

2. Die Aktivierungsventilanordnung (2107) gemäß Anspruch 1, ferner aufweisend:
ein Dichtungselement (2184), welches die Flussdurchgangsauslassöffnung (24) umgibt und neben dem Schieberventil (2192) angeordnet ist;
wobei das Dichtungselement (2184) bevorzugt synthetischen Kautschuk, welcher zumindest Buna-Kautschuk, Nitrilkautschuk, Styrol-Butadien-Kautschuk und/oder Nitril-Butadien-Kautschuk beinhaltet, aufweist.

3. Die Aktivierungsventilanordnung (2107) gemäß Anspruch 1, ferner aufweisend:
ein Abgabeelement (1812), welches in dem Schieberventil (2192) angeordnet ist und die Lumeneinlassöffnung (19) und die Lumenauslassöffnung (18) beinhaltet,
wobei das Ausgabeelement (1812) bevorzugt Polyaryletheretherketon (PEEK) aufweist.

4. Die Aktivierungsventilanordnung (2107) gemäß Anspruch 1,
wobei das Target zumindest eine Zielläsion, einen Applikator und/oder eine Fluidaufnahmevorrichtung beinhaltet, oder wobei der Fluss des kryogenen Materials (1821) durch die Ventilanordnung (2107) bei einem Durchfluss von ungefähr 0,0035 g/s liegt.

5. Die Aktivierungsventilanordnung (2107) gemäß Anspruch 1,
wobei die Flussdurchgangseinlassöffnung (29) ein Stechelement (2170) beinhaltet, wobei das Stechelement bevorzugt zumindest einen vertikalen Winkel von 1-10 Grad und/oder einen abgeschrägten Winkel von 5 bis 30 Grad beinhaltet.

6. Die Aktivierungsventilanordnung (2107) gemäß Anspruch 1, ferner aufweisend:
einen Filter (2194), welcher in dem Flussdurchgang (30) zwischen der Flussdurchgangseinlassöffnung (29) und der Flussdurchgangsauslassöffnung (24) angeordnet ist,
wobei der Filter (2194) bevorzugt ein poröses Material aufweist, welches dazu ausgebildet ist, einem Druck bis ungefähr 750-2250 psi und einer Temperatur im Bereich von ungefähr 0 °C bis ungefähr - 190 °C standzuhalten, oder
wobei der Filter (2194) bevorzugt eine Dicke im Bereich von ungefähr 0,5 mm bis ungefähr 12,0 mm aufweist, oder
wobei der Filter (2194) bevorzugt eine Porosität im Bereich von ungefähr 20% bis ungefähr 35% aufweist, oder
wobei der Filter (2194) eine Porengröße im Bereich von ungefähr 1 µm bis ungefähr 4 µm aufweist, oder
wobei der Filter (2194) bevorzugt im Wesentlichen scheibenförmig ist und einen Durchmesser im Bereich von ungefähr 1,0 mm bis ungefähr 5,0 mm aufweist.

7. Die Aktivierungsventilanordnung (2107) gemäß Anspruch 1, ferner aufweisend:
ein Dichtungselement (2171), welches die Flussdurchgangseinlassöffnung (2) umgibt, wobei das Dichtungselement (2171) bevorzugt synthetischen Kautschuk, welcher zumindest Buna-Kautschuk, Nitrilkautschuk, Styrol-Butadien-Kautschuk und/oder Nitril-Butadien-Kautschuk beinhaltet, aufweist.

8. Vorrichtung zum Abgeben von kryogenem Fluid (1910), aufweisend:
eine Aktivierungsventilanordnung (2107) gemäß Anspruch 1;
den Ventilkörper (2191), welcher dazu ausgebildet ist, in Flussverbindung mit einem ein kryogenes Fluid enthaltenden Reservoir (1835) gebracht zu werden, wobei die Flussdurchgangseinlassöffnung (29) dazu ausgebildet ist, kryogenes Material (1821) von dem Reservoir (1835) aufzunehmen; und
ein Betätigungselement (13), welches dazu ausgebildet ist, sich zwischen einer ersten Position, in welcher das Schieberventil (2192) mit dem Ventilkörper (2191) in Eingriff steht und in einer geschlossenen Position positioniert ist, bei welcher die Lumeneinlassöffnung (19) nicht fluchtet und von einer Flussverbindung mit der Flussdurchgangsauslassöffnung (24) des Ventilkörpers blockiert ist, und einer zweiten Position, in welcher das Schieberventil (2192) mit dem Ventilkörper (2191) in Eingriff steht und in einer geöffneten Position verschieblich fluchtet, bei welcher die Lumeneinlassöffnung (19) mit der Flussdurchgangsauslassöffnung (24) des Ventilkörpers (2191) fluchtet, zu bewegen, um einen Flusspfad (29, 30, 31) zu bilden, um einen Fluss des kryogenen Materials (1821) durch die Ventilanordnung (2107) zu ermöglichen,.

9. Vorrichtung zum Abgeben von kryogenem Fluid (1910) gemäß Anspruch 8, wobei
die Vorrichtung (1910) derart ausgebildet ist, dass während einer Abgabeverwendung, eine kryogene Flüssigkeit in dem Reservoir (1835) näher an der Flussdurchgangseinlassöffnung (29) ist als ein Gas in dem Reservoir (1835), oder
wobei der Ventilkörper (2191) weiterhin ein hohles Stechelement (2170) aufweist, welches ausgebildet ist, einen das kryogene Material (1821) einhausenden Behälter (1820) zu durchstechen und, um den Ventilkörper (2191) in Flussverbindung mit dem Reservoir (1835) des kryogenen Materials (1821) zu bringen, oder
wobei die Vorrichtung (1910) gemäß Anspruch 8 ferner aufweist:
einen Betätigungshebel (1802), welcher in einem Vorrichtungsgehäuse (1911) angeordnet ist und dazu ausgebildet ist, mit dem Behälter (1820) des kryogenen Materials einzugreifen, um das hohle Stechelement (2170) zu kontaktieren, um den Behälter (1820) zu durchstechen, um den Ventilkörper (2191) in Flussverbindung mit dem Reservoir (1835) des kryogenen Materials (1821) zu bringen, oder
wobei die Vorrichtung (1910) gemäß Anspruch 8 ferner aufweist:
zumindest einen Filter (2194), welcher in dem Flussdurchgang (30) angeordnet ist, wobei der zumindest eine Filter (2194) als Flussregelmechanismus zum Begrenzen eines Durchflusses von kryogenem Fluid ausgebildet ist, wenn das kryogene Fluid (1821) aus dem Reservoir (1820) zu der Lumenauslassöffnung (18) fließt.

## Revendications

1. Ensemble valve d'activation (2107) comprenant :
un corps de valve (2191) comprenant un passage d'écoulement (30) comportant une ouverture d'entrée (29) de passage d'écoulement qui reçoit une substance cryogénique (1821) et une ouverture de sortie (24) de passage d'écoulement qui amène la substance cryogénique (1821) reçue ; et
un tiroir de valve (2192) accouplé à coulissement au corps de valve (2191), le tiroir de valve (2192) comprenant une ouverture d'entrée de lumière (19) destinée à recevoir de la substance cryogénique (1821) à partir de l'ouverture de sortie (24) de passage d'écoulement et une ouverture de sortie de lumière (18) qui amène la substance cryogénique (1821) vers une cible ;
dans lequel le tiroir de valve (2192) est accouplé au corps de valve (2191) et placé dans une position fermée dans laquelle l'ouverture d'entrée de lumière (19) est décalée vis-à-vis de l'ouverture de sortie (24) de passage d'écoulement du corps de valve (2191) afin d'empêcher un écoulement de la substance cryogénique (1821) ;
dans lequel le tiroir de valve (2192) est accouplé au corps de valve (2191) et placé à coulissement dans une position ouverte dans laquelle l'ouverture d'entrée de lumière (19) est alignée sur l'ouverture de sortie (24) de passage d'écoulement du corps de valve (2191) afin de former une voie d'écoulement de façon à permettre un écoulement de la substance cryogénique (1821) à travers l'ensemble valve ; et
dans lequel le tiroir de valve (2192) comprend des rainures de clavette (2182) en renfoncement usinées pour recevoir des clavettes (2181) usinées sur le corps de valve (2191).

2. Ensemble valve d'activation (2107) selon la revendication 1, comprenant en outre :
un élément d'étanchéité (2184) entourant l'ouverture de sortie (24) de passage d'écoulement et disposé en position adjacente au tiroir de valve (2192),
dans lequel l'élément d'étanchéité (2184) comprend de préférence un caoutchouc synthétique comprenant au moins un caoutchouc parmi le caoutchouc Buna, le caoutchouc nitrile, le caoutchouc styrène-butadiène et le caoutchouc nitrile butadiène.

3. Ensemble valve d'activation (2107) selon la revendication 1, comprenant en outre :
un élément de distribution (1812) disposé dans le tiroir de valve (2192) et comprenant l'ouverture d'entrée de lumière (19) et l'ouverture de sortie de lumière (18),
dans lequel l'élément de distribution (1812) comprend de préférence de la polyaryléthe-réthercétone (PEEK).

4. Ensemble valve d'activation (2107) selon la revendication 1, dans lequel la cible comprend au moins une cible parmi une lésion cible, un applicateur et un dispositif de retenue de fluide, ou dans lequel le débit de la substance cryogénique (1821) à travers l'ensemble valve (2107) est d'environ 0,035 g/s.

5. Ensemble valve d'activation (2107) selon la revendication 1, dans lequel l'ouverture d'entrée (29) de passage d'écoulement comprend un élément de perforation (2170), dans lequel l'élément de perforation comprend de préférence un angle vertical allant de 1 à 10 degrés et/ou un angle de biseau allant de 5 à 30 degrés.

6. Ensemble valve d'activation (2107) selon la revendication 1, comprenant en outre :
un filtre (2194) disposé dans le passage d'écoulement (30) entre l'ouverture d'entrée (29) de passage d'écoulement et l'ouverture de sortie (24) de passage d'écoulement,
dans lequel le filtre (2194) comprend de préférence un matériau poreux configuré pour résister à une pression allant jusqu'à environ 750 à 2250 psi et une température allant d'environ 0 °C à environ -190 °C, ou
dans lequel le filtre (2194) présente de préférence une épaisseur allant d'environ 0,5 mm à environ 12 mm, ou
dans lequel le filtre (2194) présente de préférence une porosité allant d'environ 20 % à environ 35 %, ou
dans lequel le filtre (2194) présente une taille de pore allant d'environ 1 µm à environ 4 µm, ou
dans lequel le filtre (2194) est de préférence essentiellement en forme de disque et présente un diamètre allant d'environ 1 mm à environ 5 mm.

7. Ensemble valve d'activation (2107) selon la revendication 1, comprenant en outre :
un élément d'étanchéité (2171) entourant l'ouverture d'entrée (29) de passage d'écoulement, dans lequel l'élément d'étanchéité (2171) comprend de préférence un caoutchouc synthétique comprenant au moins un caoutchouc parmi le caoutchouc Buna, le caoutchouc nitrile, le caoutchouc styrène-butadiène et le caoutchouc nitrile butadiène.

8. Dispositif de distribution de fluide cryogénique (1910) comprenant :
un ensemble valve d'activation (2107) selon la revendication 1 ;
le corps de valve (2191) étant configuré pour être placé en communication d'écoulement avec un réservoir (1835) contenant un fluide cryogénique, l'ouverture d'entrée (29) de passage d'écoulement étant configurée pour recevoir une substance cryogénique (1821) à partir du réservoir (1835) ; et
un élément d'actionnement (13) configuré pour se déplacer entre une première position dans laquelle le tiroir de valve (2192) est accouplé au corps de valve (2191) et placé dans une position fermée dans laquelle l'ouverture d'entrée de lumière (19) n'est pas alignée sur l'ouverture de sortie (24) de passage d'écoulement du corps de valve (2191) et la communication d'écoulement avec celle-ci est bloquée et une seconde position dans laquelle le tiroir de valve (2192) est accouplé au corps de valve (2191) et aligné à coulissement dans une position ouverte dans laquelle l'ouverture d'entrée de lumière (19) est alignée sur l'ouverture de sortie (24) de passage d'écoulement du corps de valve (2191) afin de former une voie d'écoulement (29, 30, 31) de façon à permettre un écoulement de la substance cryogénique (1821) à travers l'ensemble valve (2107).

9. Dispositif de distribution de fluide cryogénique (1910) selon la revendication 8, dans lequel
le dispositif (1910) est configuré de telle sorte que, lors de son utilisation pour la distribution, un liquide cryogénique dans le réservoir (1835) se trouve plus près de l'ouverture d'entrée (29) de passage d'écoulement qu'un gaz dans le réservoir (1835), ou
dans lequel le corps de valve (2191) comprend en outre un élément de perforation creux (2170) configuré pour percer un réceptacle (1820) renfermant la substance cryogénique (1821) et pour placer le corps de valve (2191) en communication d'écoulement avec le réservoir (1835) de substance cryogénique (1821), ou
dans lequel le dispositif (1910) selon la revendication 8 comprend en outre :
un levier d'activation (1802) disposé dans un boîtier de dispositif (1911) et configuré pour accoupler avec le réceptacle de substance cryogénique (1820) de façon à ce qu'il vienne en contact avec l'élément de perforation creux (2170) afin de percer le réceptacle (1820) pour placer le corps de valve (2191) en communication d'écoulement avec le réservoir (1835) de substance cryogénique (1821), ou
dans lequel le dispositif (1910) selon la revendication 8 comprend en outre :
au moins un filtre (2194) disposé dans le passage d'écoulement (30), le ou les filtres (2194) étant configurés sous la forme d'un mécanisme de régulation d'écoulement afin de limiter un débit de fluide cryogénique tandis que le fluide cryogénique (1821) s'écoule du réservoir (1820) à l'ouverture de sortie de lumière (18).
